# EUROPEAN PATENT SPECIFICATION

(11) **EP 1 557 152 B1**
(45) Date of publication and mention of the grant of the patent: **26.01.2011**
(21) Application number: 03770032.5
(22) Date of filing: 30.10.2003
(51) Int. Cl.: A61K 8/88, A61Q 5/00, A61Q 19/00

(54) **COSMETICS EXCELLENT IN TEXTURE AND OIL-DISPERSIBILITY**
KOSMETIKA MIT HERVORRAGENDER STRUKTUR UND DISPERGIERBARKEIT
COMPOSITION COSMETIQUE AYANT UNE EXCELLENTE TEXTURE ET UNE EXCELLENTE DISPERSABILITE DANS L'HUILE

(30) Priority: 31.10.2002 JP 2002317556; 25.11.2002 JP 2002340359
(43) Date of publication of application: 27.07.2005
(73) Proprietor: Idemitsu Technofine Co. Ltd, Sumida-ku, Tokyo 130-0015 (JP)
(72) Inventor: MIYAYAMA, Tetsuo, Sumida-ku, Tokyo (JP)
(74) Representative: Gille Hrabal Struck Neidlein Prop Roos
(86) International application number: PCT/JP2003/013938
(87) International publication number: WO 2004/039339

(56) References cited:
- EP-A1- 1 152 024
- EP-A2- 0 211 223
- JP-A- 6 322 358
- JP-A- 2001 072 764
- JP-A- 2001 354 542
- JP-A- 2003 012 442
- JP-A- 2003 146 830
- KUNIOKA MASAO: 'Biseibutsu ni yoru tsukurareta poly (gamma-glutamine-san) suiyoeki no gamma sen ni yoru kakyo hanno' KOBUNSHI RONBUNSHU vol. 30, no. 10, October 1993, pages 755 - 760, XP002979110

## Description

### TECHNICAL FIELD

The present invention relates to cosmetic compositions including cosmetics such as skin cosmetic materials, hair cosmetic materials and make-up cosmetic materials, and used in toiletry application including bathing agents, etc., and more particularly to cosmetic compositions that are improved in various feelings such as smooth feeling and neat feeling while keeping a good moisture retention property

Also, the present invention relates to cosmetic compositions in which an oiliness agent is well dispersed even when no surfactant or a reduced amount of a surfactant is used therein.

### BACKGROUND ART

Skin cosmetic materials such as lotion, milky lotion, cream and pack, hair cosmetic materials such as shampoo, rinse, hair cream and hair set, and make-up cosmetic materials such as foundation, rouge and eye shadow as well as bathing agents are required to exhibit a good moisture retention effect. It is widely known that a humectant is blended in lotion, cosmetic cream, hair cream, hair rinse and bathing agents for the purposes of applications to skin or hair. In addition, various humectants have been used in skin and hair cosmetic materials such as skin lotion and cream, hair shampoo and rinse, etc., in order to prevent dry-up feeling or loose feeling while keeping a moisture, and impart a moist feeling to skin and hair.

There is known a large number of organic and inorganic substances having a high moisture absorption property. The general effect of poly-γ-glutamic acid as a moisturizer is described in JP 2001-354542 A. However, since humectants for cosmetic materials are required to have a good safety and a less adverse influence on configuration stability of products, only limited kinds of these substances are practically usable as the humectants for cosmetic materials. Further, the humectants are required to show not only a water-absorbing capability but also a capability for preventing evaporation of water. Thus, if it is intended to attain various properties mentioned above by using ordinary humectants, the humectants must be used in the form of a composite material thereof in many cases. However, the high moisture-absorptive substances such as sodium lactate are an electrolyte and, therefore, have properties undesirable for formulation of products such as emulsification-inhibiting effect, resulting in limited amount of the substances used as well as limited applications thereof. On the other hand, it is known that polyols such as glycerin, sorbitol and propylene glycol exhibit a relatively good moisture retention property. However, since products containing the polyols tend to have a stickiness, cosmetics, etc., using the polyols tend to be deteriorated in feelings upon use.

In the methods of blending polyaspartic acid or amino acid esters in cosmetic materials (refer to Japanese Patent Application Laid-Open Nos. Showa 61(1986)-33107 and Heisei 10(1998)-251402), the cosmetic materials exhibit a good moisture retention effect, but are insufficient in moist feeling, resulting in sticky feeling thereof. Thus, it has been demanded to provide cosmetic materials that are improved in feelings such as smooth feeling and neat feeling.

Also, skin cosmetic materials such as lotion, milky lotion, cream and pack, hair cosmetic materials such as shampoo, rinse, hair cream and hair set, make-up cosmetic materials such as foundation, rouge and eye shadow, as well as bathing agents are classified into an oil type and a non-oil type. The oil type cosmetic materials contain an oiliness agent dispersed therein (for example, refer to Japanese Patent Application Laid-Open No. 2000-38314).

In the case where the oiliness agent is dispersed in the cosmetic materials, a dispersant containing a synthetic material such as a nonionic surfactant as an effective ingredient has been conventionally added thereto in order to improve the dispersibility of the oiliness agent. However, the cosmetic materials containing the nonionic surfactant show irritativeness against skin. Further, from the standpoint of reduction of environmental burden, natural fats and oils have been increasingly used as the oiliness agent. However, if it is intended to attain good feelings such as smooth feeling and neat feeling, the natural fats and oils must be used at a high concentration, thereby causing problems such as stickiness of resultant products.

Under these circumstances, an object of the present invention is to provide a cosmetic composition which exhibits not only less stickiness upon use, a good familiarity with skin and hair, a good moisture retention effect and a good moist feeling, but also is improved in smooth feeling and neat feeling. Also, another object of the present invention is to provide a cosmetic composition which exhibits a less stickiness upon use and a good familiarity with skin and hair, and in which an oiliness agent is well dispersed even though no surfactant or a reduced amount of a surfactant is used therein.

### DISCLOSURE OF THE INVENTION

As a result of extensive researches for accomplishing the above objects, the inventors have found that when a crosslinked product of poly-γ-glutamic acid and/or a crosslinked product of a poly-γ-glutamic acid salt having a particle size of 0.1 to 100 µm and an average particle size of 1 to 50 µm is added as a humectant, the resultant cosmetic material can exhibit less stickiness upon use and a good moisture retention effect, and can be improved in various feelings such as smooth feeling and neat feeling. That is, in the case of ordinary cosmetic materials, if a humectant used therein has a large particle size, the cosmetic materials exhibit a good moist feeling (moisture retention property), but show a rough feeling. Whereas, if the humectant has a small particle size, the cosmetic materials exhibit a less rough feeling and a good neat feeling, but tend to be deteriorated in moisture retention property. In the present invention, it has been found that when using a crosslinked product of poly-γ-glutamic acid and/or a crosslinked product of a poly-γ-glutamic acid salt having an average particle size in the above specific range, the resultant cosmetic materials can be improved in feelings such as smooth feeling and neat feeling while keeping a good stability in an acid range as well as a good moisture retention property which are inherent to the crosslinked product of poly-γ-glutamic acid and/or the crosslinked product of a poly-γ-glutamic acid salt. The present invention has been accomplished on the basis of the above findings.

Further, the inventors have found that when the crosslinked product of poly-γ-glutamic acid and/or the crosslinked product of a poly-γ-glutamic acid salt is added as an oil dispersion modifier, the resultant cosmetic materials can exhibit less stickiness upon use and a good effect of dispersing an oiliness agent therein, in particular, are free from flaking (non-whitening effect when spreading over hair) in the case of hair cosmetic materials. The present invention has also been accomplished on the basis of the above finding.

Thus, the present invention provides the following cosmetic compositions:
[1] A cosmetic composition comprising a crosslinked product of poly-γ-glutamic acid and/or a crosslinked product of a poly-γ-glutamic acid salt having a particle size of 0.1 to 100 µm and an average particle size of 1 to 50 µm and having a gelation rate of 20 to 100%.
[2] The cosmetic composition of the aspect [1], wherein said crosslinked product of poly-γ-glutamic acid and/or said crosslinked product of a poly-γ-glutamic acid salt is contained in an amount of 0.001 to 20% by mass.
[3] The cosmetic composition of the aspect [1], wherein said crosslinked product of poly-γ-glutamic acid or said crosslinked product of a poly-γ-glutamic acid salt is produced by exposing at least one solution selected from the group consisting of an aqueous solution, a methyl alcohol solution and an ethyl alcohol solution of poly-γ-glutamic acid or the poly-γ-glutamic acid salt which contain poly-γ-glutamic acid in an amount of 2 to 30% by mass, to radiation for crosslinking thereof.
[4] The cosmetic composition of the aspect [3], wherein said radiation is γ-ray or electron beam.
[5] The cosmetic composition according to any of the aspects [1] to [4], wherein said cosmetic composition is used as hair cosmetic materials, skin cosmetic materials or nail cosmetic materials .
[6] The cosmetic composition according to any of the aspects [1] to [5], comprising an oiliness agent selected from the group consisting of vegetable oils, higher alcohols or esters thereof, higher fatty esters and liquid paraffins, and the crosslinked product as an oil dispersion modifier.
[7] The cosmetic composition of the aspect [6], wherein said oiliness agent is contained in an amount of 0.01 to 80% by mass, and said crosslinked product of poly-γ-glutamic acid and/or said crosslinked product of a poly-γ-glutamic acid salt is contained in an amount of 0.1 to 30% by mass.
[8] The cosmetic composition of the aspect [6], wherein said crosslinked product of poly-γ-glutamic acid or said crosslinked product of a poly-γ-glutamic acid salt is produced by exposing at least one solution selected from the group consisting of an aqueous solution, a methyl alcohol solution and an ethyl alcohol solution of poly-γ-glutamic acid or the poly-γ-glutamic acid salt which contain poly-γ-glutamic acid in an amount of 1 to 30 % by mass, to radiation for crosslinking thereof.
[9] The cosmetic composition of the aspect [8], wherein said radiation is γ-ray or electron beam.
[10] Use of cosmetic composition according to any of the aspects [6] to [9] as a hair cosmetic material, a skin cosmetic material or a nail cosmetic material.

### BEST MODE FOR CARRYING OUT THE INVENTION

The poly-γ-glutamic acid used as a raw material of a humectant in the cosmetic material with excellent feeling according to the present invention is not particularly limited, and there may be used those poly-γ-glutamic acids obtained by various production methods. Examples of the production methods include cultivation methods using microorganisms such as cultivation method using Bacillus subtilis, cultivation method using gene-recombination microorganisms and cultivation method controlled by Bacillus natto, and chemical synthesis methods. In the case where the poly-γ-glutamic acid is produced by the cultivation method using microorganisms, there may be used any strains capable of producing the poly-γ-glutamic acid outside of the fungus body. Of these strains, especially preferred are Bacillus funguses. Specific examples of the strains include Bacillus subtilis, Bacillus anthracis and Bacillus natto. In particular, the poly-γ-glutamic acids having a molecular weight of several millions or more which are produced by microorganisms such as Bacillus subtilis are preferably used (Japanese Patent Application Laid-Open No. Heisei 1(1989)-174397).

In the cultivation method using microorganisms, there may be used any strains or culture media as long as the poly-γ-glutamic acid is suitably produced thereby. For example, any synthetic culture media or any natural culture media may be employed as long as these culture media suitably contain a carbon source, a nitrogen source, inorganic substances and other nutrients. Examples of amino acids added to the culture media include L-glutamic acid, aspartic acid, alanine, leucine, phenyl alanine, histidine and salts thereof. Of these amino acids and salts thereof, preferred is L-glutamic acid. The amino acids or salts thereof may be added in an amount of 2 to 12% and preferably 3 to 10%.

Examples of the carbon source include glucose, sucrose, citric acid and xylose. Of these carbon sources, preferred are citric acid and glucose. Examples of the nitrogen source include organic nutrient sources such as peptone and yeast extracts, and inorganic nutrient sources such as ammonium sulfate.

The cultivation may be conducted under aerobic conditions such as shake culture and agitation culture. The culture temperature is in the range of 25 to 45°C and preferably 30 to 40°C, and the pH value of the culture media upon the cultivation is in the range of 5 to 9 and preferably 6 to 8. The pH value of the culture media upon the cultivation may be controlled by adding sodium hydroxide, potassium hydroxide, etc., thereto.

The culture time is usually in the range of 48 to 72 h in which the poly-γ-glutamic acid is accumulated outside of the fungus body. After completion of the cultivation, the poly-γ-glutamic acid in the cultivation solution may be recovered by conventionally known methods. More specifically, the fungus body is removed from the cultivation solution by centrifugal separation or filtration using a filtering assistant or a filter with micropores, and then the resultant solution is subjected to untrafiltration, thereby recovering the poly-γ-glutamic acid. Alternatively, the cultivation solution may be mixed with ethanol in an amount 3 to 4 times that of the solution to precipitate the poly-γ-glutamic acid from the solution. The resultant precipitate is dissolved in water to remove water-insoluble components therefrom. Successively, the obtained solution is subjected to dialysis, ultrafiltration, etc., to remove low-molecular weight substances therefrom, and then to repeated re-precipitation procedure with ethanol, etc., to recover the poly-γ-glutamic acid therefrom.

The poly-γ-glutamic acid salt may be obtained by known reactions using the poly-γ-glutamic acid. Examples of the poly-γ-glutamic acid salt include alkali metal salts such as sodium, potassium and lithium salts, ammonium salts, ethanol amine salts, diethanol amine salts, triethanol amine salts and basic amine salts of the poly-γ-glutamic acid.

The crosslinked product of poly-γ-glutamic acid used as a humectant in the cosmetic material with excellent feeling according to the present invention may be produced by dissolving the poly-γ-glutamic acid obtained by the above-mentioned cultivation method using microorganisms or chemical synthesis method in a solvent to prepare a solution containing the poly-γ-glutamic acid in an amount of 2 to 30% by mass, preferably 2 to 20% by mass and more preferably 5 to 15% by mass, exposing the obtained solution to radiation, and then separating and purifying the resultant crosslinked product from the solution. Examples of the solvent include water, alcohol, acetone, methyl acetate and ethyl acetate. Of these solvents, preferred are water, methyl alcohol and ethyl alcohol, and more preferred is water. The crosslinked product of the poly-γ-glutamic acid salt may be produced by the same method as described above.

The solution in which the poly-γ-glutamic acid or poly-γ-glutamic acid salt is dissolved, is placed in a radiation-permeable container, for example, a glass vial bottle, etc. The radiation usable in the present invention is not particularly limited. Examples of the radiation include α-ray, β-ray, γ-ray, electron beam, neutron and X-ray. Of these radiation rays, preferred are γ-ray and electron beam. The amount of electron beam irradiated varies depending upon conditions such as water absorptivity of the aimed crosslinked product, and is preferably 20 kGy or more. The irradiation time of less than 1 s tends to be insufficient to obtain the crosslinked product in some cases. Therefore, the irradiation time is preferably 1 s or longer.

The above method using such an electron beam irradiation enables production of the crosslinked product of poly-γ-glutamic acid or the crosslinked product of poly-γ-glutamic acid salt having a desired gelation rate. The gelation rate of the crosslinked product of poly-γ-glutamic acid or the crosslinked product of poly-γ-glutamic acid salt used as a humectant in the present invention is in the range of 20 to 100%, preferably 40 to 100% and more preferably 60 to 100%. If the gelation rate of the crosslinked product is less than 20%, the crosslinked product tends to be insufficient in moisture retention property, and the productivity thereof tends to be lowered.

Meanwhile, the gelation rate used herein means a value obtained by dividing a dried mass of the humectant composed of the crosslinked product of poly-γ-glutamic acid by the amount of the poly-γ-glutamic acid subjected to the electron beam irradiation, namely, a percentage of the dried mass of the humectant composed of the crosslinked product of poly-γ-glutamic acid based on the amount of the poly-γ-glutamic acid initially charged. The gelation rate of the crosslinked product of poly-γ-glutamic acid salt has the same meaning as described above.

Thereafter, the crosslinked product is subjected to freeze-drying, etc., to remove water therefrom, thereby obtaining the crosslinked product of poly-γ-glutamic acid or the crosslinked product of poly-γ-glutamic acid salt in the form of a solid. The crosslinked product of poly-γ-glutamic acid or the crosslinked product of poly-γ-glutamic acid salt thus obtained is colorless and transparent, and exhibits an excellent water absorption property as well as a good biodegradability.

The particle size of the crosslinked product of poly-γ-glutamic acid or the crosslinked product of poly-γ-glutamic acid salt may be controlled by subjecting the crosslinked product from which water is removed, to fine pulverization treatment using, for example, a jet mill, a roll press, a ball mill, etc. The fine pulverization treatment is preferably conducted under an environment in which a dried air is circulated, to prevent coagulation of the particles. If desired, the particle size of the crosslinked product may be controlled by ultrasonic classification, etc.

In the cosmetic material with excellent feeling according to the present invention, the crosslinked product of poly-γ-glutamic acid or the crosslinked product of poly-γ-glutamic acid salt is required to have a particle size of 0.1 to 100 µm and an average particle size of 1 to 50 µm and preferably 5 to 30 µm If the particle size of the crosslinked product of poly-γ-glutamic acid or the crosslinked product of poly-γ-glutamic acid salt is less than 0.1 µm, the resultant cosmetic material tends to be deteriorated in moisture retention property. On the other hand, if the particle size of the crosslinked product of poly-γ-glutamic acid or the crosslinked product of poly-γ-glutamic acid salt is more than 100 µm, the resultant cosmetic material tends to be deteriorated in neat feeling.

The crosslinked product of poly-γ-glutamic acid or the crosslinked product of poly-γ-glutamic acid salt which is used as a humectant in the cosmetic material with excellent feeling according to the present invention, can be widely used in lotion, milky lotion, cold cream, hand cream, rouge, eye shadow, hair spray, hair tonic, hair dressing, shampoo, rinse, perm solution, sweat remover, bathing agent, etc.

The cosmetic material with excellent feeling according to the present invention contains the crosslinked product of poly-γ-glutamic acid or the crosslinked product of poly-γ-glutamic acid salt in an amount of usually 0.001 to 20% by mass, preferably 0.001 to 10% by mass, more preferably 0.01 to 5% by mass and still more preferably 0.1 to 2.5% by mass. If the amount of the crosslinked product added is less than 0.001% by mass, the resultant cosmetic material tends to be insufficient in moisture retention effect. If the amount of the crosslinked product added is more than 20% by mass, the resultant cosmetic material tends to exhibit undesirable sticky feeling.

The crosslinked product of poly-γ-glutamic acid and/or the crosslinked product of poly-γ-glutamic acid salt which is used as a humectant in the cosmetic material with excellent feeling according to the present invention, may be used in combination with other humectants, for example, propylene glycol, sorbitol, aminic acids, sodium lactate, etc., without damage to the effects thereof.

On the other hand, the oiliness agent used in the cosmetic material having an excellent oil dispersibility according to the present invention may be selected from the group consisting of vegetable oils, higher alcohols or esters thereof, higher fatty esters and liquid paraffins. Examples of the vegetable oils include olive oil, jojoba oil, sweet almond oil, grape seed oil, rose hip oil, avocado oil, sesame oil, wheat germ oil, evening primrose oil, camellia oil and sasanqua oil.

Examples of the higher alcohols include octyl dodecanol, hexyl dodecanol and oleyl alcohol. Examples of the esters of higher alcohols include oleyl oleate, octyl stearate, butyl adipate, isocetyl octanoate, isocetyl isostearate, isononyl isononanoate, isopropyl isostearate, isostearyl isostearate, isotridecyl isononanoate, oleyl erucate, coconut oil alkyl (caprylate/caprate), neopentyl glycol dicaprate, dioctyl ether, pentaerythritol tetra-2-ethylhexanoate, pentaerythritol tetraisostearate, hexyldecanol/hexyldecyl laurate, shea fats, dioctyl cyclohexane, squarane, cetyl 2-ethylhexacenoate, cetostearyl isononanoate, trimethylolpropane trioctanoate, trimethylolpropane tristearate, isocetyl stearate and octyldodecyl myristate.

Examples of the higher fatty esters include mono-, di- or tri-esters of glycerol such as glycerol polyoxyethylene coconut oil fatty acid esters, glycerol triisopalmitate and glycerol tri(caprylate or caprate), isostearyl lactate, hexyl laurate, decyl oleate, isopropyl myristate and isopropyl palmitate.

These oiliness agents may be used singly or in the combination of any two or more thereof.

The poly-γ-glutamic acid used as a raw material of the oil dispersion modifier in the cosmetic material with an excellent oil dispersibility according to the present invention is not particularly limited, and there may be used those poly-γ-glutamic acids obtained by various production methods. Examples of the production methods include cultivation methods using microorganisms such as cultivation method using Bacillus subtilis, cultivation method using gene-recombination microorganisms and cultivation method controlled by Bacillus natto, and chemical synthesis methods. The method of producing the poly-γ-glutamic acid by the cultivation method using microorganisms may be conducted in the same manner as described above.

The crosslinked product of poly-γ-glutamic acid used as the oil dispersion modifier in the cosmetic material with an excellent oil dispersibility according to the present invention may be produced by dissolving the poly-γ-glutamic acid obtained by the above-mentioned cultivation method using microorganisms or chemical synthesis methods in a solvent to prepare a solution containing the poly-γ-glutamic acid in an amount of 1 to 30% by mass, preferably 2 to 20% by mass and more preferably 5 to 15% by mass, exposing the obtained solution to radiation in the same manner as described above, and then separating and purifying the resultant crosslinked product from the solution.

The gelation rate of the crosslinked product of poly-γ-glutamic acid or the crosslinked product of poly-γ-glutamic acid salt which is used as the oil dispersion modifier in the cosmetic material with excellent oil dispersibility according to the present invention is in the range of 20 to 100%, preferably 40 to 100% and more preferably 60 to 100%. If the gelation rate of the crosslinked product is less than 20%, the crosslinked product tends to be insufficient in moisture retention property, and the productivity thereof tends to be lowered. Meanwhile, the definition of the gelation rate is the same as described above.

The particle size of the crosslinked product of poly-γ-glutamic acid or the crosslinked product of poly-γ-glutamic acid salt may be suitably controlled by the same method as explained concerning the cosmetic material with excellent feeling.

In the cosmetic material with excellent oil dispersibility according to the present invention, from the standpoints of well-balanced properties between moisture retention property and feelings such as smooth feeling and neat feeling, the crosslinked product of poly-γ-glutamic acid or the crosslinked product of poly-γ-glutamic acid salt preferably has a particle size of 0.1 to 100 µm and an average particle size of 1 to 50 µm. The average particle size is more preferably 5 to 30 µm. If the particle size of the crosslinked product is less than 0.1 µm, the resultant cosmetic material tends to be deteriorated in moisture retention property. On the other hand, if the particle size of the crosslinked product is more than 100 µm, the resultant cosmetic material tends to be deteriorated in neat feeling.

The crosslinked product of poly-γ-glutamic acid or the crosslinked product of poly-γ-glutamic acid salt which is used as the oil dispersion modifier in the cosmetic material with excellent oil dispersibility according to the present invention, can be widely used in lotion, milky lotion, cold cream, hand cream, rouge, eye shadow, hair spray, hair tonic, after-treatment agent, hair dressing, shampoo, rinse, perm solution, sweat remover, bathing agent, etc. Further, the crosslinked product may be used as a remover for make-up cosmetic materials such as rouge, eye shadow and cheek rouge.

The remover composition for make-up cosmetic materials may contain, in addition to the above crosslinked product of poly-γ-glutamic acid and/or the crosslinked product of poly-γ-glutamic acid salt, various components used in ordinary cosmetic materials such as, for example, surfactants, oleums, solvents, gelling agents, drugs, water-swelling clay minerals, polymers, pigments, antiseptic agents, viscosity modifiers, antioxidants, perfumes and water, unless the addition thereof adversely affects the effects of the present invention.

As the surfactants, there may be used various kinds of surfactants. Specific examples of the anionic surfactants include sulfate-based and sulfonate-based surfactants such as alkyl sulfates, polyoxyethylene alkyl sulfates, sulfosuccinic acid-based surfactants, taurate-based surfactants, isethionate-based surfactants and α-olefinsulfonic acid-based surfactants, carboxylate-based surfactants such as fatty acid soaps, ethercarboxylic acid-based surfactants and acylated amino acid-based surfactants, and phosphate-based surfactants such as alkyl phosphate-based surfactants.

Specific examples of amphoteric surfactants include carbo-betaine-based surfactants, phospho-betaine-based surfactants, sulfo-betaine-based surfactants and imidazolium-betaine-based surfactants.

Specific examples of nonionic surfactants include polyoxyalkylene-addition type surfactants, polyoxypropylene/polyoxyethylene-addition type surfactants, amine oxide-based surfactants, monoethanol amide-based surfactants and diethanol amide-based surfactants, as well as polyhydric alcohol-based surfactants such as sorbitan fatty esters, polyoxyethylene hardened castor oil and fatty esters thereof, polyethylene glycol fatty esters, glycerol fatty esters, sucrose fatty esters, alkyl saccharide -based surfactants and N-polyhydroxyalkyl fatty amide-based sugars.

Of these surfactants, preferred are the nonionic surfactants from the standpoint of good emulsification dispersibility.

In the cosmetic material with excellent oil dispersibility according to the present invention, the contents of the above oiliness agent and the above crosslinked product of poly-γ-glutamic acid and/or crosslinked product of poly-γ-glutamic acid salt may be appropriately determined according to kinds of cosmetic materials. The contents of the above oiliness agent and the above crosslinked product of poly-γ-glutamic acid or crosslinked product of poly-γ-glutamic acid salt are usually 0.01 to 80% by mass and 0.1 to 30% by mass, respectively, preferably 0.05 to 30% by mass and 0.1 to 10% by mass, respectively, and more preferably 0.1 to 10% by mass and 0.1 to 5% by mass, respectively. If the amount of the crosslinked product added is less than 0.1% by mass, the resultant cosmetic material tends to be insufficient in oil dispersibility. If the amount of the crosslinked product added is more than 30% by mass, the resultant cosmetic material tends to suffer from sticky feeling.

The crosslinked product of poly-γ-glutamic acid and/or the crosslinked product of poly-γ-glutamic acid salt which is used as the oil dispersion modifier in the cosmetic material with excellent oil dispersibility according to the present invention, may be used in combination with other humectants such as propylene glycol, sorbitol, aminic acids and sodium lactate without adversely affecting the effects of the present invention. Further, the cosmetic material of the present invention may be blended with additives usable in ordinary cosmetic materials, e.g., antiseptic agents such as methyl parabene and 1,3-butylene glycol unless the addition thereof adversely affects the effects of the present invention.

Next, the present invention will be described in more detail by reference to the following examples and comparative examples. However, it should be noted that these examples are only illustrative and not intended to limit the invention thereto.

### PRODUCTION EXAMPLE 1

A 10% by mass aqueous solution of Meiji g-PGT (poly-γ-glutamic acid available from Meiji Seika Kaisha Co., Ltd.) was placed in a glass tray, and irradiated with electron beam at a intensity of 2.5 kGy/s from a distance of 10 cm for a total period of 12 s using a Cockcroft-Walton-type electron beam irradiation apparatus such that the amount of electron beam irradiated was 30 kGy. The resultant treated material was immersed in water at 4°C for one week to remove uncrosslinked poly-γ-glutamic acid therefrom. The water-swelled poly-γ-glutamic acid gel was separated by filtration through a metal screen with 80 mesh, and then freeze dried to obtain a crosslinked product of poly-γ-glutamic acid having a gelation rate of 91% and an average particle size of 200 µm. The thus obtained crosslinked product of poly-γ-glutamic acid was subjected to fine pulverization treatment using a ball mill and a jet mill, thereby obtaining particles of the crosslinked product of poly-γ-glutamic acid having an average particle size of 10 µm. The obtained particles were further subjected to fine pulverization treatment, thereby obtaining fine particles of the crosslinked product of poly-γ-glutamic acid having an average particle size of 0.1 µm.

Meanwhile, the gelation rate was determined as a percentage of a dried mass of the resultant poly-γ-glutamic acid gel to the mass of poly-γ-glutamic acid initially charged. In addition, the above average particle size was measured using a laser-type particle size distribution measuring apparatus "LMS-3" available from Seishin Kigyo Co., Ltd., utilizing a principle of Fraunhofer diffraction phenomenon.

### EXAMPLES 1 AND 2, REFERENCE EXAMPLES 1 AND 2, AND COMPARATIVE EXAMPLES 1 TO 3

The crosslinked product of poly-γ-glutamic acid obtained in PRODUCTION EXAMPLE 1 was blended with water, ethyl alcohol and a perfume at a mass ratio as shown in Table 1, thereby producing a skin cosmetic material. Meanwhile, as the crosslinked product of poly-γ-glutamic acid, in Examples 1 and 2, there was used the crosslinked product having an average particle size of 10 µm; in Reference Examples 1 and 2 and Comparative Example 1, there was used the crosslinked product having an average particle size of 200 µm (which was subjected to no fine pulverization treatment); and in Comparative Example 3, there was used the crosslinked product having an average particle size of 0.1 µm.

The above skin cosmetic material was applied to human skin and then dried to evaluate a moist feeling, a tidy feeling and a neat feeling thereof according to the following evaluation ratings. The results are shown in Table 1.
Moist feeling: A: Good; B: Poor
Tidy feeling: A: No stickiness; B: Sticky
Neat feeling: A Good; B: Poor

### COMPARATIVE EXAMPLE 4

The same procedure as in Example 1 was repeated except for using Meiji γ-PGA as an uncrosslinked poly-γ-glutamic acid instead of the crosslinked product of poly-γ-glutamic acid. The results are shown in Table 1.

**TABLE 1-1**

| | Example | | Reference Example | |
|---|---|---|---|---|
| Amount blended (% by mass) | 1 | 2 | 1 | 2 |
| Water | 89.4 | 89.0 | 89.4 | 89.0 |
| Ethyl alcohol | 10.0 | 10.0 | 10.0 | 10.0 |
| Perfume | 0.5 | 0.5 | 0.5 | 0.5 |
| Crosslinked product of poly-γ-glutamic acid | | | | |
| Average particle size: 200 µm | | | 0.1 | 0.5 |
| Average particle size: 10 µm | 0.1 | 0.5 | | |
| Average particle size: 0.1 µm | | | | |
| Uncrosslinked poly-γ-glutamic acid | | | | |
| Evaluation results | | | | |
| Moist feeling | A | A | A | A |
| Tidy feeling | A | A | A | A |
| Neat feeling | A | A | B | B |

**TABLE 1-2**

| | Comparative Example | | | |
|---|---|---|---|---|
| Amount blended (% by mass) | 1 | 2 | 3 | 4 |
| Water | 64.5 | 89.5 | 89.0 | 89.4 |
| Ethyl alcohol | 10.0 | 10.0 | 10.0 | 10.0 |
| Perfume | 0.5 | 0.5 | 0.5 | 0.5 |
| Crosslinked product of poly-γ-glutamic acid | | | | |
| Average particle size: 200 µm | 25.0 | | | |
| Average particle size: 10 µm | | | | |
| Average particle size: 0.1 µm | | | 0.5 | |
| Uncrosslinked poly-γ-glutamic acid | | | | 0.1 |
| Evaluation results | | | | |
| Moist feeling | A | B | B | B |
| Tidy feeling | B | A | A | A |
| Neat feeling | B | B | B | B |

### EXAMPLES 3 TO 7

The crosslinked product of poly-γ-glutamic acid obtained in PRODUCTION EXAMPLE 1 was blended with an oiliness agent, water and an antiseptic agent at the mass ratio as shown in Table 2, thereby producing a hair cosmetic material (after-treatment agent). Meanwhile, as the crosslinked product of poly-γ-glutamic acid, in Examples 3 to 5, there was used the crosslinked product having an average particle size of 10 µm; and in Examples 6 and 7, there was used the crosslinked product having an average particle size of 1 mm which was subjected to no fine pulverization treatment.

The above hair cosmetic material was applied to hair and then dried to evaluate an oil dispersibility, a smooth feeling, occurrence of flaking (whitening caused upon spreading over the hair) and irritativeness against skin according to the following evaluation ratings. The results are shown in Table 2. Meanwhile, the oil dispersibility was evaluated by visual observation.
Oil dispersibility: A: Good; B: Slightly good; C: Poor
Neat feeling: A Good; B: Slightly good; C: Poor
Flaking: A: None; B: Occurred
Irritativeness against skin: A: None; B: Occurred

### COMPARATIVE EXAMPLE 5

The same procedure as in Example 3 was repeated except for using Meiji γ-PGA as an uncrosslinked poly-γ-glutamic acid instead of the crosslinked product of poly-γ-glutamic acid. The results are shown in Table 2.

### COMPARATIVE EXAMPLE 6

The same procedure as in Example 3 was repeated except for using polyoxyethylene (40) hardened castor oil as a nonionic surfactant instead of the crosslinked product of poly-γ-glutamic acid. The results are shown in Table 2.

### COMPARATIVE EXAMPLE 7

The same procedure as in Example 3 was repeated except for using polyvinyl pyrrolidone instead of the crosslinked product of poly-γ-glutamic acid. The results are shown in Table 2.

### COMPARATIVE EXAMPLE 8

The same procedure as in Example 3 was repeated except for using no crosslinked product poly-γ-glutamic acid. The results are shown in Table 2.

**TABLE 2-1**

| | Example | | | | |
|---|---|---|---|---|---|
| Amount blended (% by mass) | 3 | 4 | 5 | 6 | 7 |
| Water | 91.0 | 91.0 | 91.0 | 91.0 | 91.0 |
| Cetyol ISL | 5.0 | | | 5.0 | |
| Cetyol PEEH4 | | 5.0 | | | 5.0 |
| Cetyol S | | | 5.0 | | |
| Methyl parabene | 0.2 | 0.2 | 0.2 | 0.2 | 0.2 |
| 1,3-butylene glycol | 1.8 | 1.8 | 1.8 | 1.8 | 1.8 |
| Crosslinked product of poly-γ-glutamic acid | | | | | |
| Average particle size: 10 µm | 2.0 | 2.0 | 2.0 | | |
| Average particle size: 1 mm | | | | 2.0 | 2.0 |
| Uncrosslinked poly-γ-glutamic acid | | | | | |
| NIKKOL HCO-40 | | | | | |
| PVP K-90 | | | | | |
| Evaluation results | | | | | |
| Oil dispersibility | A | A | A | B | B |
| Neat feeling | A | A | A | B | B |
| Flaking | A | A | A | A | A |
| Irritativeness against skin | A | A | A | A | A |

**TABLE 2-2**

| | Comparative Example | | | |
|---|---|---|---|---|
| Amount blended (% by mass) | 5 | 6 | 7 | 8 |
| Water | 91.0 | 91.0 | 92.0 | 93.0 |
| Cetyol ISL | 5.0 | 5.0 | 5.0 | 5.0 |
| Cetyol PEEH4 | | | | |
| Cetyol S | | | | |
| Methyl parabene | 0.2 | 0.2 | 0.2 | 0.2 |
| 1,3-butylene glycol | 1.8 | 1.8 | 1.8 | 1.8 |
| Crosslinked product of poly-γ-glutamic acid | | | | |
| Average particle size: 10 µm | | | | |
| Average particle size: 1 mm | | | | |
| Uncrosslinked poly-γ-glutamic acid | 2.0 | | | |
| NIKKOL HCO-40 | | 2.0 | | |
| PVP K-90 | | | 1.0 | |
| Evaluation results | | | | |
| Oil dispersibility | C | C | A | C |
| Neat feeling | C | C | C | C |
| Flaking | A | A | C | A |
| Irritativeness against skin | A | C | C | A |

| | | | | |
|---|---|---|---|---|
| Note: Cetyol ISL: Isostearyl lactate available from Cognis Japan Co., Ltd.; Cetyol PEEH4: Pentaerythritol tetra-2-ethylhexanoic acid available from Cognis Japan Co., Ltd.; Cetyol S: Dioctyl cyclohexane available from Cognis Japan Co., Ltd.; NIKKOL HCO-40: Polyoxyethylene (40) hardened castor oil available from Nikko Chemicals Co., Ltd.; and PVP K-90: Polyvinyl pyrrolidone available from BASF | | | | |

### EXAMPLE 8

Ninety six percents by mass of water, 2.0% by mass of a crosslinked product of poly-γ-glutamic acid having an average particle size of 30 µm, 0.2% by mass of methyl parabene and 1.8% by mass of 1,3-butanediol were uniformly dissolved and dispersed to obtain a cosmetic remover composition. Meanwhile, the crosslinked product of poly-γ-glutamic acid having an average particle size of 30 µm was produced by finely pulverizing the crosslinked product of poly-γ-glutamic acid having an average particle size of 200 µm which was obtained during the production process of Production Example 1 into those having an average particle size of 30 µm by the same method as in Production Example 1.

The thus obtained cosmetic remover composition was used for removing a rouge having the following composition which was prepared by an ordinary method, and the following evaluations were performed. The results are shown in Table 3.

| Composition of rouge: | |
|---|---|
| Glyceryl tri(caprylate /caprate) | 15.0% by mass |
| Castor oil | 32.6% by mass |
| Pentaerythrityl tetraoctanoate | 5.0% by mass |
| Microcrystalline wax | 5.0% by mass |
| Neopentyl glycol dicaprate | 5.0% by mass |
| Candelilla wax | 11.0% by mass |
| Ceresin | 5.0% by mass |
| Sucrose stearate | 3.0% by mass |
| Glycerol fatty ester | 2.0% by mass |
| Squarane | 2.0% by mass |
| Tocopherol | 2.0% by mass |
| BHT | 1.0% by mass |
| Butyl parabene | 0.1% by mass |
| Propyl parabene | 0.1% by mass |
| Citric acid | 1.0% by mass |
| Barium sulfate | 1.0% by mass |
| Titanium oxide | 2.0% by mass |
| Mica | 1.0% by mass |
| Red #202 | 2.0% by mass |
| Red #204 | 1.0% by mass |
| Red #218 | 0.5% by mass |
| Blue #204 | 1.5% by mass |
| Yellow #205 | 1.2% by mass |

### <Evaluation Method>

### (1) Cleanability

Five female panelists put the above rouge on their lips, and after the elapse of 2 h, wiped off the lips with a cotton impregnated with the above cosmetic remover composition. The cosmetic removing effect was visually observed and evaluated according to the following evaluation ratings.
A: Cleaned off very well
B: Cleaned off well
C: Most cleaned off
D: Almost uncleaned

### (2) Irritativeness against skin

Five female panelists wiped off their lips and surrounding skin thereof with a cotton impregnated with the above cosmetic remover composition. The irritativeness against skin was evaluated according to the following ratings.
A: All of the five panelists felt no irritativeness against their skins.
B: No irritativeness but some physical disorder feeling on skin
C: One or more panelists felt irritativeness against skin.

### (3) Feeling upon use

Upon evaluating the above cleanability, a touch feeling (stickiness) after using the cosmetic remover composition was evaluated according to the following evaluation ratings.
A: Neat feeling and good touch feeling
B: No stickiness
C: Slight stickiness
D: Considerable stickiness

### EXAMPLE 9

The cosmetic remover composition prepared by blending 99 parts by mass of the cosmetic remover composition obtained in Example 8 and 1 part by mass of liquid paraffin with each other was evaluated by the same method as described in Example 8. The results are shown in Table 3.

### EXAMPLE 10

The cosmetic remover composition prepared by blending 95 parts by mass of the cosmetic remover composition obtained in Example 8 and 5 parts by mass of liquid paraffin with each other was evaluated by the same method as described in Example 8. The results are shown in Table 3.

### COMPARATIVE EXAMPLE 9

Liquid paraffin was used instead of the cosmetic remover composition obtained in Example 8, and evaluated by the same method as described in Example 8. The results are shown in Table 3.

### COMPARATIVE EXAMPLE 10

A cosmetic remover composition containing the following components which was prepared by an ordinary method was used instead of the cosmetic remover composition obtained in Example 8, and evaluated by the same method as described in Example 8. The results are shown in Table 3.

### <Cosmetic Remover Composition>

| | |
|---|---|
| Liquid paraffin | 52.0% by mass |
| Isopropyl palmitate | 2.0% by mass |
| Sorbes-30 tetraoleate | 5.0% by mass |
| Sorbitan sesquioleate | 2.0% by mass |
| Olive oil | 2.0% by mass |
| Tocopherol | 2.0% by mass |
| Soybean oil | 4.0% by mass |
| Perfume | 0.5% by mass |
| Water | 30.5% by mass |

**TABLE 3**

| Evaluation items | Example | | | Comparative Example | |
|---|---|---|---|---|---|
| | 8 | 9 | 10 | 9 | 10 |
| Cleanability | B | B | A | C | A |
| Irritativeness against skin | A | A | A | C | C |
| Feeling upon use | A | A | B | D | C |

### INDUSTRIAL APPLICABILITY

In accordance with the present invention, by using a crosslinked product of poly-γ-glutamic acid and/or a crosslinked product of a poly-γ-glutamic acid salt having a particle size of 0.1 to 100 µm and an average particle size of 1 to 50 µm as a humectant, it is possible to readily produce a cosmetic material with excellent feeling which exhibits a moist feeling, a less stickiness and a neat feeling.

The crosslinked product of poly-γ-glutamic acid or the crosslinked product of poly-γ-glutamic acid salt which is used as the humectant in the cosmetic material with excellent feeling according to the present invention exhibits an excellent moisture retention effect even when added to the cosmetic material in a small amount, a good familiarity with skin and hair, and a good biodegradability, and can be, therefore, widely used in lotion, milky lotion, cold cream, hand cream, rouge, eye shadow, hair spray, hair tonic, hair dressing, shampoo, rinse, perm solution, sweat remover, bathing agent, etc.

In addition, in the present invention, when the crosslinked product of poly-γ-glutamic acid and/or the crosslinked product of poly-γ-glutamic acid salt is used as an oil dispersion modifier, it is possible to readily produce a cosmetic material having an excellent oil dispersibility, a less stickiness, a less irritativeness against skin, and a neat feeling.

The crosslinked product of poly-γ-glutamic acid or the crosslinked product of poly-γ-glutamic acid salt which is used as the oil dispersion modifier in the cosmetic material with excellent oil dispersibility according to the present invention exhibits an excellent oil dispersing effect even when added to the cosmetic material in a small amount, a good familiarity with skin and hair, a good biodegradability, and an excellent cleanability and a good touch feeling after use, as well as a less irritativeness against eyes, and can be, therefore, widely used in lotion, milky lotion, cold cream, hand cream, rouge, eye shadow, hair spray, hair tonic, after-treatment agent, hair dressing, shampoo, rinse, perm solution, sweat remover, bathing agent, etc., and further can also be used as a remover for make-up cosmetic materials such as rouge, eye shadow and cheek rouge.

## Claims

1. A cosmetic composition comprising a crosslinked product of poly-γ-glutamic acid and/or a crosslinked product of a poly-γ-glutamic acid salt having a particle size of 0.1 to 100 µm and an average particle size of 1 to 50 µm and having a gelation rate of 20 to 100%.

2. The cosmetic composition according to claim 1, wherein said crosslinked product of poly-γ-glutamic acid and/or said crosslinked product of a poly-γ-glutamic acid salt is contained in an amount of 0.001 to 20% by mass.

3. The cosmetic composition according to claim 1, wherein said crosslinked product of poly-γ-glutamic acid or said crosslinked product of a poly-γ-glutamic acid salt is produced by exposing at least one solution selected from the group consisting of an aqueous solution, a methyl alcohol solution and an ethyl alcohol solution of poly-γ-glutamic acid or the poly-γ-glutamic acid salt which contain poly-γ-glutamic acid in an amount of 2 to 30% by mass, to radiation for crosslinking thereof.

4. The cosmetic composition according to claim 3, wherein said radiation is γ-ray or electron beam.

5. The cosmetic composition according to any of claims 1 to 4, wherein said cosmetic composition is used as hair cosmetic materials, skin cosmetic materials or nail cosmetic materials .

6. A cosmetic composition according to any of claims 1 to 5, comprising an oiliness agent selected from the group consisting of vegetable oils, higher alcohols or esters thereof, higher fatty esters and liquid paraffins, and the crosslinked product as an oil dispersion modifier.

7. The cosmetic composition according to claim 6, wherein said oiliness agent is contained in an amount of 0.01 to 80% by mass, and said crosslinked product of poly-γ-glutamic acid and/or said crosslinked product of a poly-γ-glutamic acid salt is contained in an amount of 0.1 to 30% by mass.

8. The cosmetic composition according to claim 6, wherein said crosslinked product of poly-y -glutamic acid or said crosslinked product of a poly-γ-glutamic acid salt is produced by exposing at least one solution selected from the group consisting of an aqueous solution, a methyl alcohol solution and an ethyl alcohol solution of poly-γ-glutamic acid or the poly-γ-glutamic acid salt which contain poly-γ-glutamic acid in an amount of 1 to 30 % by mass, to radiation for crosslinking thereof.

9. The cosmetic composition according to claim 8, wherein said radiation is γ-ray or electron beam.

10. Use of cosmetic composition according to any of claims 6 to 9 as a hair cosmetic material, a skin cosmetic material or a nail cosmetic material.

## Patentansprüche

1. Kosmetische Zusammensetzung, umfassend ein vernetztes Produkt aus Poly-γ-Glutaminsäure und/oder ein vernetztes Produkt aus einem Poly-γ-Glutaminsäuresalz, welches eine Partikelgröße von 0,1 bis 100 µm und eine mittlere Partikelgröße von 1 bis 50 µm und eine Gelierungsrate von 20 bis 100 % aufweist.

2. Kosmetische Zusammensetzung nach Anspruch 1, wobei das vernetzte Produkt aus Poly-γ-Glutaminsäure und/oder das vernetzte Produkt aus einem Poly-γ-Glutaminsäuresalz in einer Menge von 0,001 bis 20 Gewichtsprozent enthalten ist.

3. Kosmetische Zusammensetzung nach Anspruch 1, wobei das vernetzte Produkt aus Poly-γ-Glutaminsäure oder das vernetzte Produkt aus einem Poly-y-Glutaminsäuresalz hergestellt wird, indem mindestens eine Lösung, die ausgewählt wird aus der aus einer wässrigen Lösung, einer Methylalkohollösung und einer \Ethylalkohollösung von Poly-γ-Glutaminsäure oder den Poly-γ-Glutaminsäuresalz bestehenden Gruppe, die Poly-γ-Glutaminsäure in einer Menge von 2 bis 30 Gewichtsprozent enthält, zu deren Vernetzung einer Bestrahlung ausgesetzt wird.

4. Kosmetische Zusammensetzung nach Anspruch 3, wobei die Strahlen γ-Strahlen oder Elektronenstrahlen sind.

5. Kosmetische Zusammensetzung nach einem der Ansprüche 1 bis 4, wobei die kosmetische Zusammensetzung αls Haarkosmetikmaterialien, Hautkosmetikmaterialien oder Nagelkosmetikmaterialien verwendet wird.

6. Kosmetische Zusammensetzung nach einem der Ansprüche 1 bis 5, umfassend ein Öligkeitsmittel, ausgewählt aus der aus der Pflanzenölen, höheren Alkoholen oder Estern davon, höheren Fettsäuren und flüssigen Paraffinen bestehenden Gruppe, und das vernetzte Produkt ais Öldispersionsmodifizierungsmittel.

7. Kosmetische Zusammensetzung nach Anspruch 6, wobei das Öligkeitsmittel in einer Menge von 0,01 bis 80 Gewichtsprozent enthalten ist, und das vernetzte Produkt aus Poly-γ-Glutaminsäure und/oder das vernetzte Produkt aus einem Poly-γ-Glutaminsäuresalz in einer Menge von 0,1 bis 30 Gewichtsprozent enthalten ist.

8. Kosmetische Zusammensetzung nach Anspruch 6, wobei das vernetzte Produkt aus Poly-γ-Glutaminsäure oder das vernetzte Produkt aus einem Poly-γ-Glutaminsäuresalz hergestellt wird, indem mindestens eine Lösung, die ausgewählt wird aus der aus einer wässrigen Lösung, einer Methylalkohollösung und einer Ethylalkohollösung von Poly-γ-Glutaminsäure oder dem Poly-γ-Glutaminsäuresalz bestehenden Gruppe, die Poly-γ-Glutaminsäure in einer Menge von 1 bis 30 Gewichtsprozent enthält, zu deren Vernetzung einer Bestrahlung ausgesetzt wird.

9. Kosmetische Zusammensetzung nach Anspruch 8, wobei die Strahlen γ-Strahlen oder Elektronenstrahlen sind.

10. Verwendung der kosmetischen Zusammensetzung nach einem der Ansprüche 6 bis 9 als Haarkosmetikmaterial, Hautkosmetikmaterial oder Nagelkosmetikmaterial.

## Revendications

1. Composition cosmétique comprenant un produit réticulé d'acide poly-γ-glutamique et/ou un produit réticulé d'un sel d'acide poly-γ-glutamique, présentant une taille particulaire de 0,1 à 100 µm et une taille particulaire moyenne de 1 à 50 µm et ayant un taux de gélification de 20 à 100 %.

2. Composition cosmétique selon la revendication 1, dans laquelle ledit produit réticulé d'acide poly-γ-glutamique et/ou ledit produit réticulé d'un sel d'acide poly-γ-glutamique est contenu en quantité de 0,001 à 20 % en masse.

3. Composition cosmétique selon la revendication 1, dans laquelle ledit produit réticulé d'acide poly-γ-glutamique ou ledit produit réticulé d'un sel d'acide poly-γ-glutamique est produit en exposant à un rayonnement au moins une solution choisie dans le groupe constitué d'une solution aqueuse, d'une solution d'alcool méthylique et d'une solution d'alcool éthylique de l'acide poly-γ-glutamique ou du sel d'acide poly-γ-glutamique, qui contiennent l'acide poly-γ-glutamique en quantité de 2 à 30 % en masse, pour le réticuler.

4. Composition cosmétique selon la revendication 3, dans laquelle ledit rayonnement est un faisceau de rayons γ ou un faisceau d'électrons.

5. Composition cosmétique selon l'une quelconque des revendications 1 à 4, dans laquelle ladite composition cosmétique est utilisée comme matériaux cosmétiques pour les cheveux, matériaux cosmétiques pour la peau ou matériaux cosmétiques pour les ongles.

6. Composition cosmétique selon l'une quelconque des revendications 1 à 5, comprenant un agent d'onctuosité choisi dans le groupe constitué des huiles végétales, des alcools supérieurs ou leurs esters, des esters gras supérieurs et des paraffines liquides, et le produit réticulé comme modificateur de dispersion d'huile.

7. Composition cosmétique selon la revendication 6, dans laquelle ledit agent de lubricité est contenu en quantité de 0,01 à 80 % en masse, et ledit produit réticulé d'acide poly-γ-glutamique et/ou ledit produit réticulé d'un sel d'acide poly-γ-glutamique est contenu en quantité de 0,1 à 30 % en masse.

8. Composition cosmétique selon la revendication 6, dans laquelle ledit produit réticulé d'acide poly-γ-glutamique ou ledit produit réticulé d'un sel d'acide poly-γ-glutamique est produit en exposant à un rayonnement au moins une solution choisie dans le groupe constitué d'une solution aqueuse, d'une solution d'alcool méthylique et d'une solution d'alcool éthylique d'acide poly-γ-glutamique ou du sel d'acide poly-γ-glutamique, qui contiennent l'acide poly-γ-glutamique en quantité de 1 à 30 % en masse, pour le réticuler.

9. Composition cosmétique selon la revendication 8, dans laquelle ledit rayonnement est un faisceau de rayons γ ou un faisceau d'électrons.

10. Utilisation d'une composition cosmétique selon l'une quelconque des revendications 6 à 9, comme matériau cosmétique pour les cheveux, matériau cosmétique pour la peau ou matériau cosmétique pour les ongles.
